(19) Europäisches Patentamt

European Patent Office

Office européen des brevets

(11) **EP 0 835 648 A2**

(12) **EUROPÄISCHE PATENTANMELDUNG**

(43) Veröffentlichungstag:
15.04.1998 Patentblatt 1998/16

(51) Int. Cl.$^6$: **A61K 7/06**

(21) Anmeldenummer: 97116417.3

(22) Anmeldetag: 20.09.1997

(84) Benannte Vertragsstaaten:
AT BE CH DE DK ES FI FR GB GR IE IT LI LU MC
NL PT SE

(30) Priorität: 12.10.1996 DE 19642227

(71) Anmelder:
**Wella Aktiengesellschaft
64274 Darmstadt (DE)**

(72) Erfinder:
• **Schmenger, Jürgen
64331 Weiterstadt (DE)**
• **Stein, Bernd
63768 Hösbach (DE)**
• **Flemming, Ernst
63150 Heusenstamm (DE)**

(54) **Silikonhaltiges Haarbehandlungsmittel**

(57)     Die Erfindung betrifft ein Haarbehandlungsmittel mit einem Gehalt an (A) mindestens einem Polydimethylsiloxan mit Hydroxylendgruppen, (B) mindestens einem flüchtigen, linearen oder cyclischen Polydimethylsiloxan, (C) mindestens einem Isoparaffin, wobei das Mittel wasserfrei ist oder maximal 5 Gewichtsprozent Wasser enthält.

Auf Haaren, die mit dem erfindungsgemäßen Mittel behandelt wurden, bildet sich ein glatter Schutzfilm mit brillantem Glanz, ohne das Haar zu belasten.

EP 0 835 648 A2

**Beschreibung**

Die Erfindung betrifft ein Mittel zur Behandlung, insbesondere zur Pflege und zum Schutz von Haaren.

Die Haare werden durch Einwirkungen verschiedener Art in ihren physikalischen, chemischen und morphologischen Eigenschaften negativ beeinflußt. So wird das Haar durch kosmetische Behandlungen, wie wiederholtes Bleichen, Dauerwellen und Färben, aber auch durch häufiges Waschen der Haare mit entfettenden Tensiden, durch Klimaeinflüsse wie Feuchte- und Temperaturunterschiede oder die intensive Einwirkung von Sonnenlicht sowie durch mechanische Behandlung wie Bürsten, Kämmen und Frottieren stark strapaziert und geschädigt. Das Haar wird spröde und verliert seinen Glanz.

Es sind bereits Mittel bekannt, welche das Haar schützen, pflegen oder ihm wieder Glanz verleihen. Diese Mittel weisen jedoch den Nachteil auf, daß sie das Haar gleichzeitig belasten oder einen Fettfilm auf Haut und Haar hinterlassen oder daß sie, wenn sie auf Basis von wässrigen Lösungen vorliegen, den gewünschten Pflege-, Schutz- oder Glanzeffekt nicht langanhaltend zeigen. In der US 5,089,253 ist ein Haarbehandlungsmittel beschrieben mit einem Gehalt an einem Polydiorganosiloxan mit Hydroxylendgruppen, einem Vernetzungsmittel und einem niedermolekularem Träger. Dieses Mittel weist insbesondere hinsichtlich der damit verbundenen Belastung des Haares Nachteile auf.

Es bestand daher die Aufgabe, ein Haarbehandlungsmittel zur Verfügung zu stellen, welches das Haar gleichzeitig pflegt, schützt und glänzend aussehen läßt, ohne dabei das Haar unangenehm zu belasten. Unter einer Belastung des Haares ist eine für den Anwender eines Haarbehandlungsmittels spürbare und fühlbare Anlagerung von Stoffen auf dem Haar zu verstehen, die dem Anwender den Eindruck von unsauberem Haar vermittelt.

Es wurde nun gefunden, daß die gestellte Aufgabe gelöst wird durch ein Haarbehandlungsmittel mit einem Gehalt an

(A) mindestens einem Polydimethylsiloxan mit Hydroxylendgruppen,
(B) mindestens einem flüchtigen, linearen oder cyclischen Polydimethylsiloxan,
(C) mindestens einem Isoparaffin, wobei das Mittel wasserfrei ist oder maximal 5 Gewichtsprozent Wasser enthält und vorzugsweise frei ist von Vernetzungsmitteln.

Das erfindungsgemäße Mittel enthält die Komponente (A) vorzugsweise in einer Menge von 0,01 bis 50 Gewichtsprozent, besonders bevorzugt in einer Menge von 0,1 bis 20 Gewichtsprozent. Polydimethylsiloxane mit Hydroxylendgruppen sind auch unter der Bezeichnung α-Hydroxy-ω-hydroxypoly[oxy-(dimethylsilylen)] oder unter der CTFA-Bezeichnung Dimethiconol bekannt (CTFA International Cosmetic Ingredient Dictionary, USA 1991). Geeignete Dimethiconole werden beispielsweise gelöst in cyclischem oder linearem Polydimethylsiloxan von der Firma Dow Corning/USA unter den Handelsnamen Dow Corning Q2-1401 und Q2-1403 oder von der Firma Goldschmidt AG, Essen/Deutschland unter den Handelsnamen Abil® OSW 12 und Abil® OSW 13 vertrieben.

Das erfindungsgemäße Mittel enthält die Komponente (B) vorzugsweise in einer Menge von 0,1 bis 80 Gewichtsprozent, besonders bevorzugt in einer Menge von 5 bis 50 Gewichtsprozent. Lineare und cyclische Polydimethylsiloxane sind auch unter den CTFA-Bezeichnungen Dimethicone beziehungsweise Cyclomethicone bekannt. Beispiele für geeignete flüchtige, lineare Polydimethylsiloxane sind Polydimethylsiloxane mit einer Viskosität von 0,1 bis 20.000 mm$^2$·s$^{-1}$, vorzugsweise mit einer Viskosität von 0,5 bis 500 mm$^2$·s$^{-1}$, zum Beispiel die Handelsprodukte Dow Corning 200 Fluid und 225 Fluid der Firma Dow Corning/USA oder Abil® 5000 und Abil® 10000 der Firma Goldschmidt/Deutschland oder Belsil® DMC Typen der Firma Wacker/Deutschland.

Beispiele für geeignete flüchtige, cyclische Polydimethylsiloxone sind Cyclomethicone mit 4 bis 6 Siliziumatomen oder deren Gemische, zum Beispiel die Handelsprodukte Dow Corning 244 Fluid und Dow Corning 245 Fluid der Firma Dow Corning/USA, Abil® K4 der Firma Goldschmidt/Deutschland oder GE Silicone SF 1202 und 1173 der Firma GE Silicones/USA.

Das erfindungsgemäße Mittel enthält die Komponente (C) vorzugsweise in einer Menge von 0,1 bis 80 Gewichtsprozent, besonders bevorzugt in einer Menge von 5 bis 50 Gewichtsprozent. Geeignete Isoparaffine sind verzweigte Kohlenwasserstoffe mit 4 bis 20, besonders bevorzugt mit 6 bis 12 Kohlenstoffatomen oder deren Gemische zum Beispiel Isodekan, Isododekan oder das Handelsprodukt Solvent ID der Firma BP/Großbritannien.

Das erfindungsgemäße Mittel enthält maximal 5 Gewichtsprozent Wasser oder ist wasserfrei und kann 0,1 bis 99,9, vorzugsweise von 10 bis 50 Gewichtsprozent Alkohol enthalten. Als Alkohol kommen hierbei insbesondere die für kosmetische Zwecke üblicherweise verwendeten niederen Alkohole mit 1 bis 4 Kohlenstoffatomen wie zum Beispiel Ethanol und Propanol in Betracht.

Das Haarpflegemittel gemäß der vorliegenden Erfindung kann zusätzlich diejenigen Bestandteile enthalten, die in Haarpflegemitteln üblicherweise eingesetzt werden, insbesondere Parfümöle in einer Menge von vorzugsweise 0,01 bis 5 Gewichtsprozent; Trübungsmittel wie zum Beispiel Ethylenglykoldistearat, in einer Menge von vorzugsweise 0,01 bis 5 Gewichtsprozent; Netzmittel oder Emulgatoren aus den Klassen der anionischen, kationischen, amphoteren oder nichtionogenen oberflächenaktiven Substanzen ohne Waschwirkung wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 bis 30 Gewichtsprozent; ferner Pflanzenöle und -extrakte, Feuchthaltemittel, Farbstoffe, Lichtschutzmittel, Antioxidantien, Glanzgeber und Konservierungsstoffe in einer Menge von vorzugsweise 0,01 bis 10 Gewichtsprozent.

Das erfindungsgemäße Mittel kann in verschiedenen Applikationsformen Anwendung finden, wie beispielsweise als Flüssigfester oder in Aerosolzubereitungen als Schaum oder als Spray, des weiteren als Non-Aerosol, welches mittels einer Pumpe oder als "Pump and Spray" zum Einsatz kommt.

Wenn das erfindungsgemäße Mittel in Form eines Aerosol-Sprays vorliegt, so enthält es zusätzlich 15 bis 85 Gewichtsprozent, bevorzugt 25 bis 75 Gewichtsprozent, eines Treibmittels und wird in einem Druckbehälter abgefüllt. Als Treibmittel sind beispielsweise niedere Alkane wie zum Beispiel n-Butan, i-Butan und Propan, oder auch deren Gemische mit Dimethylether sowie ferner bei den in Betracht kommenden Drücken gasförmig vorliegende Treibmittel wie beispielsweise $N_2$, $N_2O$ und $CO_2$ sowie Gemische der vorstehend genannten Treibmittel geeignet.

Das erfindungsgemäße Mittel kann auch in Form eines mit Hilfe einer geeigneten mechanisch betriebenen Sprühvorrichtung versprühbaren Non-Aerosol-Sprays vorliegen. Unter mechanischen Sprühvorrichtungen sind solche Vorrichtungen zu verstehen, welche das Versprühen einer Flüssigkeit ohne Verwendung eines Treibmittels ermöglichen. Als geeignete Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters beim Öffnen des Sprühventils kontinuierlich abgegeben wird, verwendet werden.

Unter Haarbehandlung soll die Behandlung des menschlichen Kopfhaares vor allem zum Zweck der Herstellung einer Frisur oder zur Pflege der Haare verstanden werden.

Auf Haaren, die mit dem erfindungsgemäßen Mittel behandelt wurden, bildet sich ein glatter Schutzfilm mit brillantem Glanz, ohne das Haar zu belasten. Die flüchtigen Silikonpolymere und die flüchtigen Isoparaffine vermitteln einen guten Griff und machen das Haar während der Behandlung gut frisier- und kämmbar. Nach der kurzen Frisierzeit verflüchtigen sich diese Komponenten und es bleibt ein Pflege- und Glanzfilm auf dem Haar zurück.

Die nachfolgenden Beispiele sollen den Gegenstand der Erfindung näher erläutern.

**Beispiel 1: Glanzspray mit UV-Schutz**

| | |
|---|---|
| 30,0 g | Polydimethylsiloxan (Viskosität= 0,65 mm$^2$·s$^{-1}$) |
| 6,0 g | α-Hydroxy-ω-hydroxy-poly[oxy-(dimethylsilylen)], 12prozentige Lösung in Cyclomethicon (Abil® OSW 12 der Firma Goldschmidt/Deutschland) |
| 0,1 g | p-Methoxyzimtäsure-2-ethyl-hexylester |
| 25,0 g | Isomerengemisch von Isododekanen (Solvent ID der Firma BP/Großbritannien) |
| 38,9 g | Isopropanol |
| 100,0 g | |

**Beispiel 2: Haarschutzfilm gegen Austrocknen**

| | |
|---|---|
| 45,0 g | Polydimethylsiloxan (Viskosität = 5000 mm$^2$·s$^{-1}$) |
| 10,0 g | α-Hydroxy-ω-hydroxy-poly[oxy-(dimethylsilylen)], 13prozentige Lösung in Cyclomethicon (Q2-1403 Fluid der Firma Dow Corning/USA) |
| 35,0 g | Isomerengemisch von Isododekanen |
| 10,0 g | Isopropanol |
| 100,0 g | |

**Beispiel 3: Parfüm-Glanzspray**

| | |
|---|---|
| 35,0 g | Octamethylcyclotetrasiloxan |
| 4,0 g | α-Hydroxy-ω-hydroxy-poly[oxy-(dimethylsilylen)], 13prozentige Lösung in Cyclomethicon (Abil® OSW 13 der Firma Goldschmidt/Deutschland) |
| 1,0 g | Parfüm |
| 25,0 g | Isomerengemisch von Isododekanen |
| 35,0 g | Ethanol |
| 100,0 g | |

**Beispiel 4: Haarschneide-Lotion**

| | |
|---|---|
| 30,00 g | Decamethylcyclopentasiloxan |
| 0,50 g | α-Hydroxy-ω-hydroxy-poly[oxy-(dimethylsilylen)], 12prozentige Lösung in Cyclomethicon |
| 0,25 g | Hexadecyltrimethylammoniumchlorid, 50%ige Lösung in Isopropanol/Wasser= 2,3:1 |
| 0,10 g | Parfüm |
| 40,00 g | Isomerengemisch von Isododekanen |
| 29,15 g | Ethanol |
| 100,00 g | |

Sämtliche angegebenen Prozentzahlen stellen Gewichtsprozente dar, sofern nichts anderes angegeben ist.

**Patentansprüche**

1. Haarbehandlungsmittel mit einem Gehalt an

   (A) mindestens einem Polydimethylsiloxan mit Hydroxylendgruppen,

   (B) mindestens einem flüchtigen, linearen oder cyclischen Polydimethylsiloxan,

   (C) mindestens einem Isoparaffin, wobei das Mittel wasserfrei ist oder maximal 5 Gewichtsprozent Wasser enthält.

2. Haarbehandlungsmittel nach Anspruch 1, dadurch gekennzeichnet, daß das Polydimethylsiloxan mit Hydroxylendgruppen in einer Menge von 0,01 bis 50 Gewichtsprozent enthalten ist.

3. Haarbehandlungsmittel nach einem der Ansprüche 1 oder 2, dadurch gekennzeichnet, daß das flüchtige, lineare oder cyclische Polydimethylsiloxan in einer Menge von 0,1 bis 80 Gewichtsprozent enthalten ist.

4. Haarbehandlungsmittel nach einem der Ansprüche 1 bis 3, dadurch gekennzeichnet, daß das Isoparaffin in einer Menge von 0,1 bis 80 Gewichtsprozent enthalten ist.

5. Haarbehandlungsmittel nach einen der Ansprüche 1 bis 4, dadurch gekennzeichnet, daß das flüchtige, lineare oder cyclische Polydimethylsiloxan eine Viskosität von 0,1 bis 20.000 $mm^2 \cdot s^{-1}$ aufweist.

6. Haarbehandlungsmittel nach einem der Ansprüche 1 bis 5, dadurch gekennzeichnet, daß als flüchtiges, cyclisches Polydiemthylsiloxan Cyclooctamethyltetrasiloxan enthalten ist.